# EUROPÄISCHE PATENTSCHRIFT

(11) **EP 3 062 687 B1**
(45) Veröffentlichungstag und Bekanntmachung des Hinweises auf die Patenterteilung: **14.04.2021**
(21) Anmeldenummer: 14796727.7
(22) Anmeldetag: 26.10.2014
(51) Int. Cl.: A61B 5/00, A61B 5/0205, A61B 5/024, A61B 5/1455

(54) **ELASTISCHER SENSOR ZUR MESSUNG VON VITALPARAMETERN IM GEHÖRGANG**
ELASTIC SENSOR FOR MEASURING VITAL PARAMETERS IN THE AUDITORY CANAL
CAPTEUR ÉLASTIQUE POUR LA MESURE DES PARAMÈTRES VITAUX DANS LE CONDUIT AUDITIF

(30) Priorität: 30.10.2013 DE 102013222131
(43) Veröffentlichungstag der Anmeldung: 07.09.2016
(73) Patentinhaber: Cosinuss GmbH, 81379 München (DE)
(72) Erfinder: KREUZER, Johannes, 81379 München (DE)
(74) Vertreter: Franke, Dirk
(86) Internationale Anmeldenummer: PCT/EP2014/072929
(87) Internationale Veröffentlichungsnummer: WO 2015/062999

(56) Entgegenhaltungen:
- DE-A1- 19 831 361
- DE-A1-102011 081 815

## Beschreibung

Zur Messung von Vitalparametern, wie beispielsweise der Temperatur, der Pulsfrequenz oder der Sauerstoffsättigung, im Gehörgang eines Menschen oder eines Tieres eignet sich ein Gehörgangsensor. Solche Sensoren werden in einer vorgegebenen Richtung getragen, d.h. ein Sensor für das rechte Ohr kann nur rechts getragen werden und ein Sensor für das linke Ohr kann nur links getragen werden. Gute Messergebnisse können dann geliefert werden, wenn ein guter Halt des Sensors am Ohr bzw. im Ohr gewährleistet ist. Bei optischen Messverfahren, beispielsweise bei der Pulsoximetrie oder bei der Pulsfrequenzmessung, wird eine sehr gute Positionierung benötigt, welche sich beim Tragen des Sensors nicht oder nur geringfügig verändert.

Der Sensorelementaufsatz ist üblicherweise lichtundurchlässig. Außerdem ist bekannt, dass Lichtemitter und Lichtempfänger so weit wie möglich voneinander entfernt, am besten 180° versetzt, auf dem Sensorelementaufsatz angebracht werden. Daher werden üblicherweise schwarze Sensorelementaufsätze aus Silikon verwendet. Es ist bekannt, dass ein schwarzer Sensorelementaufsatz mit 180° versetztem Lichtemitter und Lichtempfänger eine hohe Modulationstiefe erzielt.

Bei der optischen Erfassung der Pulsfrequenz oder der arteriellen Sauerstoffsättigung mittels Pulsoximetrie wird das Gewebe mit Licht durchstrahlt. Das Licht wird durch das arterielle Blut moduliert, sodass auf die Pulsfrequenz bzw. auf die Sauerstoffsättigung geschlossen werden kann.

Für die gleichzeitige Messung der Temperatur und anderer Parameter, welche mittels optischer Komponenten durchgeführt werden, ist die Anordnung der einzelnen Sensorelemente nicht trivial. Lichtemitter strahlen nicht nur Licht in das Gewebe ein, sondern erzeugen durch Stromfluss auch Wärme. Diese Wärme darf eine gewisse Verlustleistung nicht übersteigen, damit der Benutzer die Erwärmung nicht bemerkt. Auch darf die Wärme bei einer gleichzeitigen Temperaturmessung diese nicht verfälschen.

Die Offenlegungsschrift DE 198 31 361 A1 offenbar einen am Ohr anzubringenden Ohrhörer enthaltend ein Lautsprechergehäuse, um einen Lautsprecher am Eingang des Gehörgangs eines Ohrs eines Benutzers zu positionieren.

Die Offenlegungsschrift DE 10 2011 081 185 A1 offenbart einen Sensor zur Messung eines Vitalparameters im Gehörgang eines Menschen oder eines Tieres und ein Verfahren zur Bestimmung gewebsoptischer Größen, wie in der Präambel vom Anspruch 1 beschrieben.

Es ist die Aufgabe der Erfindung, einen Sensor bereitzustellen, der dazu geeignet ist, den Benutzer nicht in seiner Mobilität einzuschränken, angenehm beim Tragen zu sein, einen optimalen Sitz zu gewährleisten und auch an verschiedene anatomische Gegebenheiten anpassbar zu sein. Ferner soll der Sensor für beide Ohren eines Menschen oder eines Tieres anpassbar sein und eine gute Signalqualität bei hoher Modulationstiefe erzielen.

Diese Aufgabe wird dadurch gelöst, dass ein Sensor zur Messung eines Vitalparameters im Gehörgang eines Menschen oder eines Tieres gemäß dem Anspruch 1 bereitgestellt wird.

Vorzugsweise wird der äußere Gehörgang als Messort herangezogen, der sich durch die Kombination von physiologischen Eigenschaften einerseits mit mechanischen, technischen und/oder funktionellen Merkmalen andererseits gerade für die mobile bzw. die kontinuierliche Sensorik eignet. Der Ausdruck, dass ein Vitalparameter gemessen wird, bedeutet, dass wenigstens ein, d.h. auch zwei oder mehrere Parameter gleichzeitig gemessen werden können. Der Ausdruck, dass das Sensorelement verbindbar mit dem Sensorelementaufsatz ist, bedeutet, dass das Sensorelement mit dem Sensorelementaufsatz, insbesondere über weitere Komponenten des Sensors oder auch über Leitungen, Drähte, Kabel oder dergleichen, verbunden werden kann. Das Sensorelement kann aber auch in direktem Kontakt mit dem Sensorelementaufsatz stehen, d.h. direkt an dem Sensorelementaufsatz angebracht sein. Vorzugsweise umfasst der Sensorelementaufsatz das Sensorelement, d.h. das Sensorelement ist als ein Teil des Sensorelementaufsatzes ausgestaltet. Der Ausdruck, dass die Position des Sensorelements im Gehörgang durch die Eindringtiefe des Sensorelementaufsatzes in dem Gehörgang bestimmt wird, bedeutet, dass die Positionierung des Sensorelements relativ zum Sensorelementaufsatz erfolgt, so dass eine genaue und kontinuierliche Messung des Vitalparameters erfolgen kann, insbesondere sind Sensorelement und Sensorelementaufsatz nahe zueinander positioniert, so dass eine Signalübertragung zwischen Sensorelementaufsatz und Sensorelement einwandfrei funktionieren kann. Vorzugsweise sind Positionierungselement und Sensorelementaufsatz einstückig ausgebildet.

Unter dem Ausdruck "Sensorelement" wird der Sensor, also der eigentliche Messfühler, verstanden, der die eigentliche physiologische bzw. biologische Größe in ein elektrisches Signal umwandelt, beispielsweise ein Wandler, ein Halbleiter, ein Messfühler oder Elektroden. Unter dem Ausdruck "Sensorelementaufsatz" und "Positionierungselement" werden funktionell und/oder mechanisch wirkverbundene Vorrichtungen verstanden, die zusammen das Sensor-element relativ zur Gehörgangswand derart positionieren, dass der physiologische bzw. biologische Parameter möglichst störungsarm gemessen werden kann. Aufgabe des Sensorelementaufsatzes ist es, die radiale Position des Sensorelements zu definieren, während es Aufgabe des Positionierungselements ist, die axiale Position zu definieren. Gemäß anderen bevorzugten Ausführungsbeispielen der Erfindung sind beide Funktionen nicht trennbar bzw. sie verschmelzen miteinander. Der Ausdruck "Sensor" steht insbesondere für die Kombination aus Sensorelement, Sensorelementaufsatz und Positionierungselement. Gemäß anderen bevorzugten Ausführungsbeispielen der Erfindung ist mit dem Ausdruck **"Sensor" auch die Auswerteeinheit gemeint, die vorzugsweise hinter dem Ohr sitzt** und dazu geeignet ist, dem Sensor Halt zu geben.

Vorzugsweise umfasst der Vitalparameter mindestens einen physiologischen, biochemischen und/oder bioelektrischen Parameter, insbesondere ist der Vitalparameter aus der Gruppe ausgewählt umfassend die Körpertemperatur, die Sauerstoffsättigung des Blutes, die Herzfrequenz, einen herzelektrischen Parameter, die Atemfrequenz, die Konzentration von im Blut gelöster Substanzen, insbesondere der arteriellen Sauerstoffsättigung, die Konzentration von im Gewebe vorhandenen Substanzen, die körperliche Aktivität und die Körperlage.

Somit sind erfindungsgemäß das Sensorelement, der Sensorelementaufsatz und das Positionierungselement wenigstens teilweise in einem Gehäuse angeordnet und das Gehäuse ist an die Anatomie wenigstens eines Teiles des Kopfes des Menschen oder des Tieres anpassbar. Damit wird der Benutzer nicht in seiner Mobilität eingeschränkt oder gestört und der Sensor ist angenehm beim Tragen und gewährleistet einen optimalen Sitz. Ferner ist der Sensor an verschiedene anatomische Gegebenheiten anpassbar, insbesondere ist er für beide Ohren eines Menschen oder eines Tieres anpassbar.

Mit "Mobilität" ist nicht nur die Beweglichkeit gemeint sondern auch eine drahtlose Übertragung der ermittelten Daten beispielsweise per Funk. Gängige Funkprotokolle wie Bluetooth oder ZigBee arbeiten entweder in den weltweit zugelassenen 2,4 bis 2,5 GHz-Bändern oder dem ISM-Band, d.h. bei 868 MHz in Europa bzw. bei 912 MHz in den USA.

Erfindungsgemäß ist also der Sensor anpassbar, vorzugsweise anatomisch anpassbar, und weist vorzugsweise ein flexibles Teil auf, welches zwei starre Teile verbindet und sich somit wenigstens teilweise an die Anatomie des Kopfes des Menschen oder des Tieres anpasst. Dazu zählt der Abstand der Ohrmuschel im oberen Bereich des Kopfes, dort wo die Ohrmuschel an den Kopf anwächst, zum Gehörgang sowie der Winkelbereich dazwischen. Durch den flexiblen bzw. elastischen Teil ist das Anlegen des Sensors angenehmer bzw. das Anlegen des Sensors an den Kopf wird dadurch erst ermöglicht.

Mit dem Wort "verdrehbar" ist gemeint, dass das Gehäuse nach rechts oder links vom Kopf des Menschen oder des Tieres aus gesehen derart gedreht bzw. verstellt werden kann, dass es an die anatomischen Gegebenheiten anpassbar ist. Die Elastizität des flexiblen Teils sorgt für einen optimalen Halt und besitzt somit eine definierte Rückstellkraft. Durch die Elastizität wird der Sensor stabil an der Ohrmuschel gehalten. Vorzugsweise wird die Verdrehung nicht durch das elastische Gehäuse realisiert, sondern durch eine Kolbenführung.

**Durch die Drehung in eine vom Kopf aus gesehen "positive" Richtung kann der** Sensor somit für eine Seite des Kopfes angepasst werden, durch die Drehung in **eine "negative"** Richtung für die andere Seite. Vorteilhafterweise wird die Verklemmung von Kolben und Führung durch ein elastisches und durch ein festes Material bereitgestellt. So wird durch die Verformung des elastischen Kolbens eine vordefinierte Kraft entstehen, sodass sich die Größenverstellung und auch Richtungsadaption beim normalen Gebrauch nicht verstellen können. Vorzugsweise umfasst das Material für den festen Teil Kunststoff und das Material für den flexiblen Teil umfasst wenigstens eines aus Gummi, Silikon und Elastomer. Beide Materialien halten vorteilhafterweise harten Witterungsbedingungen wie Salzwasser, Kälte, Sonneneinstrahlung sowie der Kontakt zur Haut, Schweiß usw. stand. Vorzugsweise weist der flexible bzw. elastische Teil einen geringen Abrieb auf. Gemäß anderen bevorzugten Ausführungsbeispielen umfasst der Kolben ein festes oder unelastisches Material und die Führung umfasst ein elastisches Material.

Gemäß einem anderen bevorzugten Ausführungsbeispiel der Erfindung legt das Gehäuse die Länge des Positionierungselements fest, besonders vorzugsweise beträgt die Länge des **Positionierungselements ≥ 10 mm und/oder ≤ 50** mm. Hierdurch erfolgt eine optimale Anpassung des Positionierungselements für den Fall einer Verwendung durch einen Menschen. Die Länge des Positionierungselementes kann im Falle der Verwendung durch ein Tier auch von diesem Längenbereich abweichen, ohne dass hierbei von dem erfinderischen Konzept der Erfindung abgewichen wird.

Gemäß einem anderen bevorzugten Ausführungsbeispiel der Erfindung ist der Sensorelementaufsatz steckbar und/oder austauschbar ausgestaltet. Somit ist der Sensorelementaufsatz leicht austauschbar und adaptierbar, was besonders vorteilhaft ist, denn der Durchmesser des Gehörgangs bei verschiedenen Personen oder Tieren kann variieren. Der Sensorelementaufsatz ist vorzugsweise in verschiedenen Größen vorhanden. Damit das Sensorelement angeschlossen werden kann ist vorteilhafterweise sowohl eine mechanische feste Verbindung als auch eine elektrische Verbindung vorhanden.

Gemäß einem anderen bevorzugten Ausführungsbeispiel der Erfindung ist der Sensorelementaufsatz wenigstens teilweise weiß ausgestaltet. Üblicherweise ist ein weißer Sensorelementaufsatz bei Lichtwellenlängen von 400 nm bis 1000 nm teilweise lichtdurchlässig, aber durch eine Anordnung der optischen Komponenten um 90° verdreht, wird effektiv Shuntlicht verhindert und eine sehr gute Messung mit hohen Modulationstiefen ermöglicht. Dadurch wird eine gute Signalqualität erhalten, denn die Modulationstiefe wird erhöht, was einem hohen Signal-Rauschverhältnis gleichkommt. Somit erzielt der erfindungsgemäße Sensor eine gute Signalqualität bei hoher oder optimaler Modulationstiefe. Bei gleichzeitiger Messung von Temperatur und Pulsfrequenz bzw. bei der Pulsoximetrie umfasst die erfindungsgemäße Anordnung die verschiedenen Sensorelemente, wobei das Sensorelement, welches die Temperatur erfasst, weiter im Gehörgang sitzt, und die optischen Sensorelemente eher weiter außen am Eingang des Gehörgangs zu sitzen kommen. Ferner muss bei der Anordnung darauf geachtet werden, dass der Temperaturfühler und der Lichtemitter oder die Lichtemitter möglichst weit voneinander entfernt sind, damit es nicht zu Störungen in der Temperaturmessung durch die Verlustleistung der Lichtemitter kommt. Eine 180°-Verdrehung von Temperaturfühler und Lichtemitter und ein Versatz, d.h. ein Temperaturfühler weiter innen und der Lichtemitter weiter außen, stellt den größtmöglichen Abstand dar.

Gemäß einem anderen bevorzugten Ausführungsbeispiel der Erfindung ist eine Schutzkappe vorgesehen, die auf dem Sensorelementaufsatz aufsetzbar ist. Vorzugsweise ist die Schutzkappe transluzent im Bereich einer Wellenlänge ≥ 400 **nm und/oder ≤ 1000 nm und/oder weist eine Materialdicke ≥ 0,005 mm und/oder ≤** 1 mm auf. Die Schutzkappe ist besonders aus hygienischen Gründen vorteilhaft, wenn es wenigstens teilweise über den Sensorelementaufsatz gezogen wird. Vorteilhafterweise kann die Schutzkappe für den Einmalgebrauch verwendet werden. Die Schutzkappe ist vorzugsweise dünn und umfasst ein flexibles Material, sodass die Messung nicht behindert wird.

Gemäß einem anderen bevorzugten Ausführungsbeispiel der Erfindung ist eine Antenne vorgesehen, die im Sensor, besonders vorzugsweise im Positionierungselement, wenigstens teilweise integrierbar ist. Besonders bevorzugt ist die Antenne komplett im Sensor integriert, so dass die Anordnung platzsparend bleibt. Für typische Datenübertragungen werden geeignete Antennen mit geeigneter Länge benötigt. Dabei verhält sich die Länge der Antenne indirekt proportional zur verwendeten Funkfrequenz. Eine gängige λ/4-Antenne ist bei 868 MHz ca. 8,6 mm lang. Vorteilhafterweise wird diese Länge im Sensor untergebracht ohne die Größe des Sensors zu vergrößern. Besonders vorzugsweise ist die Antenne im Positionierungselement wenigstens teilweise integriert.

Nachfolgend wird die Erfindung anhand eines bevorzugten Ausführungsbeispiels unter Bezugnahme auf die Zeichnung weiter im Detail erläutert.

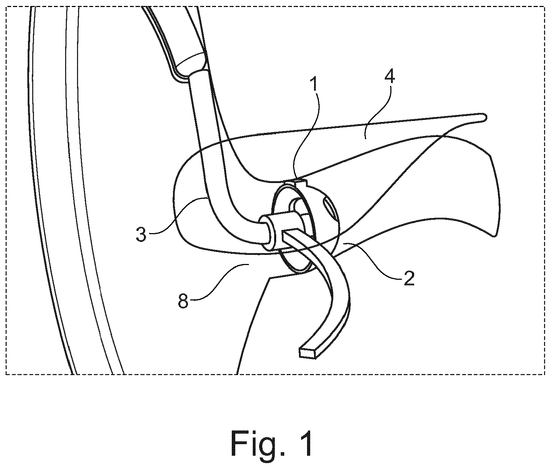

- Fig. 1: zeigt einen Sensor gemäß einem ersten bevorzugten Ausführungsbeispiel der Erfindung.

Fig. 1 zeigt einen Sensor gemäß einem ersten bevorzugten Ausführungsbeispiel der Erfindung. Dabei ist aus Fig. 1 zu erkennen, dass das Positionierungselement 3, das Sensorelement 1 und der Sensorelementaufsatz 2 in einem Gehäuse 4 angeordnet sind und das Gehäuse 4 an die Anatomie des Kopfes des Menschen anpassbar ist. Der Sensorelementaufsatz 2 weist eine weiße Farbe auf. Hierbei ist die Lichtundurchlässigkeit nicht essenziell. Der Lichtweg wird durch die Reflexion erweitert. Um eine optimale Positionierung zwischen Energie und Messsignal zu erreichen und dabei das Shuntlicht konstruktiv zu verhindern ist eine 90°-Positionierung erforderlich. Das Gehäuse 4 ist teilweise starr und teilweise flexibel ausgestaltet. Gemäß diesem bevorzugten Ausführungsbeispiel ist das Gehäuse 4 verdrehbar und größenverstellbar ausgestaltet. Weiter ist der Sensorelementaufsatz 2 austauschbar ausgestaltet.

Gemäß anderen bevorzugten Ausführungsbeispielen der Erfindung sind Sensorelementaufsätze mit verschieden farbigen Silikonschirmen vorgesehen. Der Intensitätsgrad der weißen Farbe der Sensorelementaufsätze beeinflusst dabei die Signalqualität. Erfindungsgemäß wurde festgestellt, dass je weißer der Sensorelementaufsatz ist, desto besser ist die Signalqualität.

## Patentansprüche

1. Sensor zur Messung eines Vitalparameters im Gehörgang (8) eines Menschen oder eines Tieres, umfassend:
einen Sensorelementaufsatz (2), der wenigstens teilweise im Gehörgang (8) positionierbar ist,
ein Sensorelement (1), das verbindbar mit dem Sensorelementaufsatz (2) ist und wenigstens teilweise im Gehörgang (8) positionierbar ist, und
ein Positionierungselement (3), geeignet zum Positionieren des Sensorelementaufsatzes (2) in den Gehörgang (8), wobei das Positionierungselement (3) an wenigstens einem Ende mit dem Sensorelementaufsatz (2) verbindbar ist,
wobei die Position des Sensorelements (1) im Gehörgang (8) durch die Eindringtiefe des Sensorelementaufsatzes (2) in dem Gehörgang (8) bestimmt wird, und
wobei das Positionierungselement (3) eine geeignete, die Anatomie der Ohrinnenseite nachvollziehende Formgebung aufweist, und zum Drücken des Sensorelementaufsatzes (2) in das Innere des Gehörgangs (8) geeignet ist, derart, dass der Sensor stabil gehalten wird und eine kontinuierliche Messung des Vitalparameters im Gehörgang (8) erfolgt
wobei das Sensorelement (1), der Sensorelementaufsatz (2) und das Positionierungselement (3) wenigstens teilweise in einem Gehäuse (4) angeordnet sind und das Gehäuse (4) an die Anatomie wenigstens eines Teiles des Kopfes des Menschen oder des Tieres anpassbar ist,
**dadurch gekennzeichnet, dass**
das Gehäuse (4) wenigstens teilweise elastisch ausgestaltet ist, wobei das Gehäuse (4) verdrehbar und größenverstellbar ausgestaltet ist, und
wobei das Gehäuse (4) einen Kolben und eine Führung umfasst, wobei der Kolben wenigstens teilweise in der Führung ausgerichtet ist, wobei der Kolben die Größenverstellbarkeit gewährleistet, womit sich die Länge des Positionierungselements (3) auf beliebige Größen adaptieren lässt, und
wobei der Kolben und/oder die Führung ein Material umfassend ein Kunststoff, ein Gummi, ein Silikon und/oder ein Elastomer aufweist/ aufweisen, und wobei der Kolben und die Führung in einer 0°-Position größenverstellbar sind, wobei die 0°-Position hierbei anatomisch die Richtung nach vorne oder nach hinten vom Kopf der Person oder des Tieres aus gesehen ist, wobei eine Verdrehung des Kolbens in Bezug auf die Führung um mehr als +120° bzw. -120°C nicht möglich ist, wobei durch eine Verdrehung aus der 0°-Position heraus es zu einer Verklemmung kommt, wobei in der Verklemmung eine Größenverstellbarkeit nicht möglich ist, wobei die Verklemmung von Kolben und Führung durch ein elastisches und durch ein festes Material oder durch zwei feste Materialien bereitgestellt wird.

2. Sensor nach Anspruch 1, wobei der Sensorelementaufsatz (2) steckbar und/oder austauschbar ausgestaltet ist.

3. Sensor nach einem der vorhergehenden Ansprüche, wobei der Sensorelementaufsatz (2) wenigstens teilweise weiß ausgestaltet ist.

4. Sensor nach einem der vorhergehenden Ansprüche, wobei eine Schutzkappe vorgesehen ist, die auf dem Sensorelementaufsatz (2) aufsetzbar ist.

5. Sensor nach Anspruch 4, wobei die Schutzkappe transluzent im Bereich einer Wellenlänge ≥ 400 nm und/oder ≤ 1000 nm ist und/oder eine Materialdicke ≥ 0,005 mm und/oder ≤ 1 mm aufweist.

6. Sensor nach einem der vorhergehenden Ansprüche, wobei eine Antenne vorgesehen ist, die im Sensor, vorzugsweise im vorzugsweise im Positionierungselement (3), wenigstens teilweise integrierbar ist.

## Claims

1. Sensor for measuring a vital parameter in the auditory canal (8) of a person or animal, comprising:
a sensor element attachment (2), which can be positioned at least partially in the auditory canal (8),
a sensor element (1), which can be connected to the sensor element attachment (2) and can be positioned at least partially in the auditory canal (8), and
a positioning element (3), suitable for positioning the sensor element attachment (2) in the auditory canal (8), wherein the positioning element (3) can be connected at least at one end to the sensor element attachment (2),
wherein the position of the sensor element (1) in the auditory canal (8) is determined by the depth of penetration of the sensor element attachment (2) into the auditory canal (8), and
wherein the positioning element (3) has a suitable shape that follows the anatomy of the inside of the ear, and is suitable for pushing the sensor element attachment (2) into the interior of the auditory canal (8) in such a way that the sensor is held in a stable manner and a continuous measurement of the vital parameter takes place in the auditory canal (8),
wherein the sensor element (1), the sensor element attachment (2) and the positioning element (3) are arranged at least partially in a housing (4) and the housing (4) can be adapted to the anatomy of at least part of the head of the person or animal,
**characterized in that**
the housing (4) is configured to be at least partially elastic, wherein the housing (4) is configured to be rotatable and size-adjustable, and wherein the housing (4) comprises a piston and a guide, wherein the piston is at least partially aligned in the guide, wherein the piston ensures the size-adjustability, whereby the length of the positioning element (3) can be adapted to any size, and wherein the piston and/or the guide comprise(s) a material comprising a plastic, a rubber, a silicone and/or an elastomer, and wherein the piston and the guide are size-adjustable in a 0° position, wherein the 0° position here is anatomically the forward or rearward direction as seen from the head of the person or animal, wherein a rotation of the piston relative to the guide by more than +120° or -120° is not possible, wherein a rotation out of the 0° position results in a deadlock, wherein in the deadlock a size-adjustability is not possible, wherein
the deadlock of the piston and guide is provided by an elastic material and by a rigid material or by two rigid materials.

2. Sensor according to claim 1, wherein the sensor element attachment (2) is configured to be able to be plugged on and/or replaced.

3. Sensor according to any one of the preceding claims, wherein the sensor element attachment (2) is configured to be at least partially white.

4. Sensor according to any one of the preceding claims, wherein a protective cap is provided, which can be placed onto the sensor element attachment (2).

5. Sensor according to claim 4, wherein the protective cap is translucent in the wavelength range ≥ 400 nm and/or ≤ 1000 nm, and/or has a material thickness ≥ 0.005 mm and/or ≤ 1 mm.

6. Sensor according to any one of the preceding claims, wherein an antenna is provided, which can be at least partially integrated in the sensor, preferably in the preferably in the positioning element (3).

## Revendications

1. Capteur pour la mesure d'un paramètre vital dans le conduit auditif (8) d'une personne ou d'un animal, comprenant :
un enveloppe d'élément de capteur (2), laquelle peut être mise en place au moins partiellement dans le conduit auditif (8),
un élément de capteur (1), lequel peut être raccordé à l'enveloppe d'élément de capteur (2) et peut être mis en place au moins partiellement dans le conduit auditif (8), et
un élément de mise en place (3), adapté au positionnement de l'enveloppe d'élément de capteur (2) dans le conduit auditif (8), ledit élément de mise en place (3) pouvant être raccordé à l'enveloppe d'élément de capteur (2) à au moins une extrémité, la position de l'élément de capteur (1) dans le conduit auditif (8) étant déterminée par la profondeur de pénétration de l'enveloppe d'élément de capteur (2) dans le conduit auditif (8), et
l'élément de mise en place (3) présentant une forme adéquate, reproduisant l'anatomie de l'intérieur de l'oreille, et adaptée à l'enfoncement de l'enveloppe d'élément de capteur (2) à l'intérieur du conduit auditif (8), de manière à maintenir le capteur de manière stable et à réaliser une mesure continue du paramètre vital dans le conduit auditif (8),
l'élément de capteur (1), l'enveloppe d'élément de capteur (2) et l'élément de mise en place (3) étant disposés au moins partiellement dans un boîtier (4) et le boîtier (4) étant ajustable à l'anatomie d'au moins une partie de la tête de la personne ou de l'animal,
**caractérisé en ce que**
le boîtier (4) est au moins partiellement élastique, le boîtier (4) étant conçu de manière à être rotatif et de grandeur réglable, et le boîtier (4) comprenant un piston et un guidage, le piston étant au moins partiellement centré dans le guidage, le piston assurant le réglage de grandeur, la longueur de l'élément de mise en place (3) étant ajustable à une dimension quelconque, et le piston et/ou le guidage présentant un matériau comprenant une matière plastique, un caoutchouc, une silicone et/ou un élastomère, et le piston et le guidage étant de grandeur réglable dans une position à 0°, la position à 0° étant anatomiquement la direction vers l'avant ou vers l'arrière de la tête de la personne ou de l'animal de la personne ou de l'animal, une rotation du piston de plus de +120° ou -120° étant impossible par rapport au guidage, une rotation à partir de la position à 0° entraînant un serrage, le réglage de grandeur n'étant pas possible en cas de serrage, le serrage du piston et du guidage étant réalisé par un matériau élastique et un matériau solide, ou par deux matériaux solides.

2. Capteur selon la revendication 1, où l'enveloppe d'élément de capteur (2) est prévue insérable et/ou remplaçable.

3. Capteur selon l'une des revendications précédentes, où l'enveloppe d'élément de capteur (2) est prévue au moins partiellement blanche.

4. Capteur selon l'une des revendications précédentes, où un capuchon de protection est prévu, lequel peut être mis en place sur l'enveloppe d'élément de capteur (2).

5. Capteur selon la revendication 4, où le capuchon de protection est translucide dans une plage de longueur d'onde ≥ 400 nm et/ou ≤ 1000 nm et/ou présente une épaisseur de matériau ≥ 0,005 mm et/ou ≤ 1 mm.

6. Capteur selon l'une des revendications précédentes, où une antenne est prévue, laquelle est préférentiellement au moins partiellement intégrable dans le capteur, préférentiellement dans l'élément de mise en place (3).
